(19) Europäisches Patentamt
European Patent Office
Office européen des brevets

(11) Veröffentlichungsnummer: **0 020 300**
**A1**

# (12) EUROPÄISCHE PATENTANMELDUNG

(21) Anmeldenummer: **80810168.7**

(22) Anmeldetag: **22.05.80**

(51) Int. Cl.³: **C 07 C 91/30**, A 61 K 31/135

(30) Priorität: **28.05.79 CH 4943/79**

(43) Veröffentlichungstag der Anmeldung: **10.12.80**
**Patentblatt 80/25**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Kühnis, Hans, Dr., Lange Gasse 26, CH-4052 Basel (CH)**
Erfinder: **Fuhrer, Walter, Dr., Munzackerweg 11, CH-4402 Frenkendorf (CH)**

## (54) Aromatische Hydroxyverbindungen, Verfahren zu ihrer Herstellung, pharmazeutische Präparate enthaltend solche Verbindungen und Verwendung von letzteren.

(57) Die Erfindung betrifft neue aromatische Hydroxyverbindungen der Formel

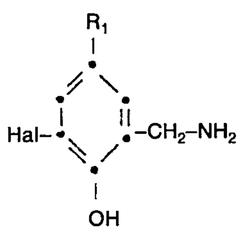

(I),

worin $R_1$ einen cycloaliphatischen Kohlenwasserstoffrest bedeutet, und Hal für Halogen steht, sowie Salze, insbesondere pharmazeutisch annehmbare nicht-toxische Säureadditionssalze solcher Verbindungen, Verfahren zu deren Herstellung, pharmazeutische Präparate, die solche Verbindungen enthalten, sowie deren Verwendung. Die neuen Verbindungen können als Diuretika und Saluretika, z.B. zur Behandlung von Oedemen, außerdem aber auch der Hypertonie verwendet werden.

EP 0 020 300 A1

CIBA-GEIGY AG 4-12371/+

Basel (Schweiz)

Aromatische Hydroxyverbindungen, Verfahren zu ihrer Herstellung, pharmazeutische Präparate enthaltend solche Verbindungen und Verwendung von letzteren.

Die Erfindung betrifft neue aromatische Hydroxyverbindungen, Verfahren zu ihrer Herstellung, pharmazeutische Präparate, die die neuen Verbindungen enthalten sowie ihre Verwendung.

Die Erfindung betrifft insbesondere neue aromatische Hydroxyverbindungen der Formel

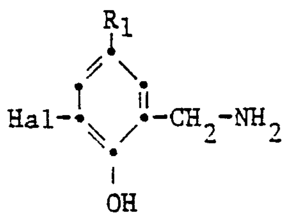

(I),

worin $R_1$ einen cycloaliphatischen Kohlenwasserstoffrest bedeutet, und Hal für Halogen steht, sowie Salze, insbesondere pharmazeutisch verwendbare, nicht-toxische Säureadditionssalze solcher Verbindungen.

Cycloaliphatische Kohlenwasserstoffreste sind gegebenenfalls niederalkylierte mono- oder poly-, wie bi- oder tricyclische Cycloalkyl- oder Cycloalkenylreste mit 3-10 Ringgliedern, wie gegebenenfalls niederalkyliertes Cyclopropyl, wie Cyclopropyl oder 1-Methylcyclopropyl, Cyclobutyl, wie Cyclobutyl oder 1-Methyl- oder 2-Methylcyclobutyl, Cyclopentyl, wie Cyclopentyl oder 1-Methyl-, 2-Methyl-, oder 3-Methyl, oder 1,3-Dimethylcyclopentyl, Cyclohexyl, wie Cyclohexyl oder 1-Methyl-, 2-Methyl-, 3-Methyl- oder 4-Methylcyclohexyl, oder 2,4-Dimethyl- oder 2,6-Dimethylcyclohexyl, 4-Aethylcyclohexyl, 4-Isopropylcyclohexyl, Cycloheptyl, wie Cycloheptyl oder 1-Methylcycloheptyl, ferner gegebenenfalls niederalkyliertes Norbornanyl, wie Norbornanyl oder 4-Methylnorbornanyl, ferner Adamantyl, z.B. 1-Adamantyl.

Gegebenenfalls niederalkylierte Cycloalkenylreste sind insbesondere gegebenenfalls niederalkylierte mono- oder poly, -wie bi- oder tricyclische 1-Cycloalkenylreste, wie Cyclopentenyloder 1-Cyclopentenyl, z.B. 1-Cyclopentenyl oder 2-Methyl- oder 3-Methyl-1-cyclopentenyl, Cyclohexenyl, wie 1-Cyclohexenyl, z.B. 1-Cyclohexenyl oder 2-Methyl-, 3-Methyl- oder 4-Methyl-1-cyclohexenyl, Cycloheptenyl wie 1-Cycloheptenyl,z.B. 1-Cycloheptenyl oder 2-Methyl- oder 3-Methyl-1-cycloheptenyl, ferner Norbornen-1-yl, wie 2- oder 5-Norbornen-1-yl z.B. 4-Methyl-2-norbornen-1-yl.

Niederalkyl enthält vorzugsweise bis und mit 7, und in erster Linie bis und mit 4 Kohlenstoffatome und ist z.B. Methyl, Aethyl, n-Propyl, Isopropyl, n-Butyl, Isobutyl, sek.-Butyl, oder tert.-Butyl, ferner n-Pentyl, n-Hexyl, Isohexyl oder n-Heptyl.

Halogen steht für Fluor und insbesondere für Chlor, Brom oder Jod.

Salze von Verbindungen der Formel I sind in erster Linie Säureadditionssalze, und insbesondere pharmazeutisch verwendbare, nicht-toxische Säureadditionssalze mit geeigneten anorganischen Säuren, wie Chlorwasserstoffsäure, Bromwasserstoffsäure, Schwefelsäure oder Phosphorsäure, oder mit geeigneten organischen aliphatischen, cycloaliphatischen, aromatischen, araliphatischen oder heterocyclischen Carbon- oder Sulfonsäuren, wie Ameisensäure, Essigsäure, Propionsäure, Bernsteinsäure, Glycolsäure, Milchsäure, Aepfelsäure, Weinsäure, Zitronensäure, Ascorbinsäure, Maleinsäure, Fumarsäure, Brenztraubensäure, Benzoesäure, Anthranilsäure, 4-Hydroxybenzoesäure, Salicylsäure, Phenylessigsäure, Embonsäure, Methansulfonsäure, Aethansulfonsäure, Hydroxyäthansulfonsäure, Aethylensulfonsaure, 4-Chlorbenzolsulfonsäure, Toluolsulfonsäure, Naphthalinsulfonsäure, Sulfanilsäure oder Cyclohexylaminsulfonsäure.

Infolge der engen Beziehungen zwischen den neuen Verbindungen in freier Form und in Form ihrer Salze sind unter den freien Verbindungen und unter den Salzen sinn- und zweckgemäss gegebenenfalls auch die entsprechenden Salze bzw. freien Verbindungen zu verstehen.

Die neuen Verbindungen besitzen wertvolle pharmakologische Eigenschaften, die insbesondere in einer starken diuretischen sowie natriuretischen und chloruretischen Wirksamkeit bestehen. Die Kaliumausscheidung wird weniger stark als die Natriumausscheidung erhöht. Diese Effekte können nach peroraler Verabreichung solcher Verbindungen in Form einer Suspension in Tragacanthschleim mittels Schlundsonde in Dosen von 10 bis 100mg/kg an Ratten und von 0,3 bis 5 mg/kg an Hunden beobachtet werden. Die Wirkungen an der Ratte können durch die Menge des während drei Stunden nach Verabreichung der Substanz ausgeschiedenen Urins im Vergleich zu einer unbehandelten Kontrollgruppe bestimmt werden, während die Wirksamkeit am Hund durch die während 5 Stunden nach Verabreichung ausgeschiedene, in Abständen von jeweils einer Stunde gesammelte Menge Urin im Vergleich zu der während einer Stunde vor Substanzgabe ausgeschiedenen Menge ermittelt werden kann.

Aufgrund dieser Eigenschaften können die neuen Verbindungen als Diuretika und Saluretika z.B.zur Behandlung von Oedemen, ausserdem aber auch der Hypertonie verwendet werden.

Die Erfindung betrifft insbesondere Verbindungen der Formel I, worin $R_1$ einen gegebenenfalls niederalkylierten Cycloalkyl- oder Cycloalkenylrest mit 5-7 Ringgliedern bedeutet und demnach für gegebenenfalls niederalkyliertes Cyclopentyl, z.B. Cyclopentyl

oder 1-Methyl- oder -2-Methylcyclopentyl, oder Cyclopentenyl, insbesondere 1-Cyclopentenyl z.B. 1-Cyclopentenyl oder 2-Methyl- oder 3-Methyl-1-cyclopentenyl, Cyclohexyl, z.B. Cyclohexyl oder 1-Methyl-, 2-Methyl-, 3-Methyl- oder 4-Methyl- oder 2,4- oder 2,6-Dimethylhexyl, 4-Aethyl- oder 4-Isopropylhexyl, Cyclohexenyl wie 1-Cyclohexenyl, z.B. 1-Cyclohexenyl oder 2- oder 3- oder 4-Methyl-1-cyclohexenyl, Cycloheptyl, wie Cycloheptyl oder 1-Methylcycloheptyl, Cycloheptenyl wie 1-Cycloheptenyl z.B. 1-Cycloheptenyl oder 2-Methyl-1-cycloheptenyl steht, und Hal für Halogen steht, oder deren Salze, insbesondere pharmazeutisch verwendbare nicht-toxische Säureadditionssalze.

Die Erfindung betrifft in erster Linie Verbindungen der Formel I, worin $R_1$ einen gegebenenfalls niederalkylierten Cycloalkyl- oder Cycloalkenylrest mit 5-6 Ringgliedern bedeutet, in dem Niederalkylreste bis und mit 4 Kohlenstoffatomen haben und demnach für gegebenenfalls niederalkyliertes Cyclopentyl, z.B. Cyclopentyl oder 1-Methyl-, 2-Methyl- oder 3-Methylcyclopentyl, oder Cyclopentenyl, insbesondere 1-Cyclopentenyl, z.B. 1-Cyclopentenyl oder 2- oder 3-Methyl-1-cyclopentenyl, Cyclohexyl, z.B. Cyclohexenyl oder 1-, oder 2-, oder 3- oder 4-Methyl- oder 2,4- oder 2,6-Dimethyl-, 4-Aethyl- oder 4-Isopropylcyclohexyl, oder Cyclohexenyl, insbesondere 1-Cyclohexenyl, z.B. 1-Cyclohexenyl oder 2- oder 3- oder 4-Methyl-1-cyclohexenyl steht, und Hal für Chlor, Brom oder Jod steht, oder deren Salze, insbesondere pharmazeutisch verwendbare nicht-toxische Säureadditionssalze.

Die Erfindung betrifft vor allem Verbindungen der Formel I, worin $R_1$ einen gegebenenfalls niederalkylierten Cyclopentyl-, Cyclopentenyl- oder Cyclohexylrest bedeutet, in dem Niederalkylreste bis und mit 3 Kohlenstoffatome haben, und demnach für gegebenenfalls niederalkyliertes Cyclopentyl, z.B. Cyclopentyl oder 1-Methyl-,

2-Methyl- oder 3-Methylcyclopentyl, oder Cyclopentenyl, insbesondere 1-Cyclopentenyl, oder 2- oder 3-Methyl-1-cyclopentenyl oder Cyclohexyl z.B. Cyclohexyl oder 1-Methyl-, 2-Methyl-, 3-Methyl- oder 4-Methyl- oder 2,4- oder 2,6-Dimethyl-, oder 4-Isopropylcyclohexyl und Hal für Brom oder Jod steht, oder deren Salze, insbesondere pharmazeutisch verwendbare nicht-toxische Säureadditionssalze.

Insbesondere betrifft die Erfindung die in den Beispielen beschriebenen Verbindungen der Formel I oder deren Salze, insbesondere pharmazeutisch verwendbare nicht-toxische Säureadditionssalze.

Die neuen Verbindungen der Formel I können nach an sich bekannten Methoden hergestellt werden.

So kann man diese erhalten, indem man z.B. in einer Verbindung der Formel

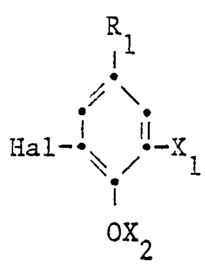

(II),

worin $X_2$ Wasserstoff ist und $X_1$ einen in die Gruppe der Formel $-CH_2NH_2$ (Ia) überführbaren Rest oder $X_1$ und $X_2$ zusammen einen unter Bildung der Hydroxygruppe und der Gruppe Ia abspaltbaren Rest darstellen, oder in einem Salz davon, den Rest $X_1$ in die Gruppe Ia überführt, oder die Reste $X_1$ und $OX_2$ gleichzeitig in die Gruppe Ia bzw. die Hydroxygruppe umwandelt, und wenn erwünscht, eine erhaltene Verbindung der Formel I in einen anderen Endstoff der Formel I umwandelt, und/oder eine erhaltene freie Verbindung in ein Salz, oder ein erhaltenes Salz in die freie Verbindung überführt, und/oder ein erhaltenes Racemat in die optischen Antipoden auftrennt.

In der Form von Salzen verwendbare Ausgansstoffe werden in erster Linie in der Form von Säureadditionssalzen, insbesondere von entsprechenden Salzen mit anorganischen Säuren, z.B. Mineralsäuren, sowie von organischen Säuren verwendet.

Ein in die Gruppe der Formel Ia überführbarer Rest ist z.B. ein mittels Reduktion, einschliesslich Hydrogenolyse, oder durch Solvolyse einschliesslich Hydrolyse, Acidolyse oder Alkoholyse in die Gruppe der Formel Ia überführbarer Rest.

Ein mittels Reduktion, einschliesslich Hydrogenolyse, in die Gruppe der Formel Ia überführbarer Rest ist z.B. eine Gruppe der Formel $-CH_2-NHX_3$ (IIa) oder der Formel $-(C=Y)-NHX'_3$ (IIb), worin Y den Oxo- oder Thioxorest darstellt, und $X'_3$ Wasserstoff oder einen unter den Bedingungen der Reduktion abspaltbaren und durch Wasserstoff ersetzbaren Rest bedeutet und $X_3$ einen unter den Bedingungen der Reduktion abspaltbaren und durch Wasserstoff ersetzbaren Rest darstellt.

Eine hydrogenolytisch abspaltbare Gruppe $X_3$ bezw. $X'_3$ ist in erster Linie eine $\alpha$-Arylniederalkylgruppe, wie eine gegebenenfalls substituierte 1-Phenylniederalkylgruppe, worin Niederalkyl bis zu 7 Kohlenstoffatome hat und worin Substituenten, insbesondere des Phenylteils, z.B. Niederalkyl oder Niederalkoxy mit jeweils bis zu 7 Kohlenstoffatomen, z.B. Methyl oder Methoxy, sein können, und ganz besonders Benzyl. Eine durch $X_2$ und $X_3$ oder $X'_3$ bzw. dem in der nachfolgenden Formel IIc definierten $X_4$ zusammen gebildete hydrogenolytisch abspaltbare Gruppe ist z.B. gegebenenfalls substituiertes 1-Phenylniederalkyliden, wobei Niederalkyliden bis zu 7 Kohlenstoffatome hat, und worin Substituenten, insbesondere des Phenylteils z.B. Niederalkyl, wie Methyl, oder Niederalkoxy, wie Methoxy, sein können, und insbesondere Benzyliden.

Insbesondere geeignet zur Reduktion von Verbindungen der Formel II mit einer Gruppe der Formel IIa oder IIb sind Hydridreduktionsmittel, wie z.B. Diboran, Natriumborhydrid, Natriumcyanoborhydrid oder Lithiumaluminiumhydrid.

Insbesondere für die Hydrogenolyse von Verbindungen der Formel II mit einer Gruppe der Formel IIa und IIb, worin $X_3'$ verschieden von Wasserstoff ist, geeignet ist katalytisch aktivierter Wasserstoff, wie Wasserstoff in Gegenwart eines geeigneten Hydrierkatalysators, z.B. eines Nickel-, Platin- oder Palladiumkatalysators.

Gruppierungen der Formel IIb, worin Y jeweils eine Thioxogruppe bedeutet, werden durch reduktive Entschwefelung, z.B. durch Behandeln mit einem Hydrierkatalysator, wie Raney-Nickel, in die Gruppierung der Formel Ia umgewandelt. Durch Beschränkung der Menge Reduktionsmittel bzw. des eingesetzten Katalysators und geeignete Wahl der Reduktionsbedingungen ist dafür zu sorgen, dass aromatisch gebundenes Halogen nicht abgespalten wird.

Weitere mittels Reduktion einschliesslich Hydrogenolyse in die Gruppe der Formel Ia überführbare Reste sind solche der Formeln $-CH=NX_4$ (IIc), -CH=NOH (IId) oder $-C\equiv N$ (IIe), worin $X_4$ einen hydrogenolytisch abspaltbaren durch Wasserstoff ersetzbaren Rest, z.B. wie oben für $X_3$ angegeben, bedeutet. Die Reduktion von Verbindungen der Formel II mit den Resten der Formel IIc einschliesslich der Abspaltung der Gruppe $X_4$, oder mit den Resten der Formeln IId oder IIe kann z.B. durch Behandeln mit katalytisch aktiviertem Wasserstoff, wie Wasserstoff in Gegenwart eines geeigneten Hydrierkatalysators z.B. eines Nickel-, Platin- oder Palladium-, aber auch eines Rhodium- oder Rutheniumkatalysators erfolgen. Oder man arbeitet mit einem geeigneten Hydridreduktionsmittel, wie Diboran oder einem Alkalimetallborhydrid, z.B. Natriumborhydrid oder Natriumcyanoborhydrid. Die Reduktion eines Ausgangsstoffes der Formel II mit der Gruppe der Formel IId kann

ausserdem mittels eines Alkalimetalls, z.B. Natrium, in einem Niederalkanol, etwa Aethanol, durchgeführt werden.

Weitere mittels Reduktion in die Gruppe der Formel Ia überführbare Reste sind Reste der Formeln $-CH_2N_3$ (IIf) oder $-CH_2NO_2$ (IIg), von denen die Gruppe der Formel IIf mittels katalytisch aktiviertem Wasserstoff , wie Wasserstoff in Gegenwart eines Hydrierkatalysators , z.B. wie oben beschrieben, oder

mittels eines Leichtmetallhydrids, z.B. Lithiumaluminiumhydrid, zur Gruppe der Formel Ia reduziert ,wird. während die Gruppe der Formel IIg z.B. mittels katalytisch aktiviertem Wasserstoff, etwa wie oben beschrieben, oder mit nascierendem Wasserstoff, z.B. mittels eines geeigneten Metalls, wie Eisen oder gegebenenfalls amalgamiertem Zink in einer Säure, z.B. einer Mineralsäure,wie wässeriger Salzsäure, zur Gruppe der Formel Ia reduziert wird. Die Reduktion der Gruppe der Formel IIf kann auch mittels eines Leichtmetallhydrids, etwa Lithiumaluminiumhydrid, erfolgen.

Unter reduktiv abspaltbaren Resten $X_3$ bezw. $X'_3$ und/oder $X_4$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl, wie 2,2,2-Trichloräthoxycarbonyl, die z.B. beim Behandeln mit einem reduzierenden Schwermetall, wie Zink, oder mit einem reduzierenden Schwermetallsalz, wie einem Chrom(II)-salz, z.B. -chlorid oder -acetat, üblicherweise in Gegenwart einer organischen Carbonsäure, wie Ameisensäure oder Essigsäure, und von Wasser abgespalten werden können.

Die obigen Reduktionsreaktionen werden in an sich bekannter Weise, üblicherweise in Gegenwart eines inerten Lösungsmittels, und, wenn notwendig, unter Kühlen oder Erwärmen, z.B. in einem Temperaturbereich von etwa -20° bis etwa +150° , und/oder in einem geschlossenen Gefäss unter Druck und/oder in einer Inertgas-, z.B. Stickstoffatmosphäre, durchgeführt.

Soweit gegebenenfalls vorhandene ungesättigte Reste $R_1$ unter den Bedingungen der beschrieben Reduktionsmethoden der Umwandlung in gesättigte Reste $R_1$ zugänglich sind, reduziert man gewünschtenfalls ungesättigte Reste $R_1$ gleichzeitig mit der reduktiven Umwandlung eines in die Gruppe Ia überführbaren Restes $X_1$, oder eines durch $X_1$ und $X_2$ zusammen gebildeten Restes unter gleichzeitiger Bildung der Hydroxygruppe und der Gruppe Ia, zu gesättigten Resten $R_1$.

Ein mittels Solvolyse wie Hydrolyse, Acidolyse oder Alkoholyse in die Gruppe der Formel I überführbarer Rest $X_1$ ist z.B. eine Gruppe der Formel $-CH_2NX_5X'_5$ (IIh), worin $X_5$ Wasserstoff und $X'_5$ ebenso wie eine solvolytisch wie hydrolytisch, acidolytisch oder alkoholytisch spaltbare und durch Wasserstoff ersetzbare Gruppe $X_2$, insbesondere einen Acylrest, wie den Acylrest einer organischen Carbonsäure, z.B. Niederalkanoyl, wie Acetyl, Halogenniederalkanoyl, wie Halogenacetyl, z.B. Choracetyl, oder Carbamoyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebenen Bedeutungen haben, und in erster Linie Trityl, darstellt, oder $X_5$ und $X'_5$ zusammen einen zwei-

basischen Acylrest, z.B. Phthaloyl bedeuten. Eine auf diese Weise abspaltbare Gruppe $X_2$ kann ausserdem Niederalkyl, z.B. Methyl, oder eine 1-Phenylniederalkylgruppe wie gegebenenfalls, z.B. wie oben definiert, substituiertes Benzyl sein. Mittels Solvolyse wie Hydrolyse, Acidolyse oder Alkoholyse in die Gruppe Ia unter gleichzeitiger Bildung der Hydroxygruppe überführbare Reste sind ferner durch $X_2$ und $X'_5$ zusammen gebildete Reste, z.B. hydrolytisch abspaltbaren Gruppen, wie Niederalkyliden, z.B. Methylen oder Isopropyliden, oder 1-Phenyl-niederalkyliden, dessen Phenylteil gegebenenfalls durch Niederalkyl, wie Methyl, oder Niederalkoxy, wie Methoxy, substituiert ist, insbesondere Benzyliden oder Cycloalkyliden, z.B. Cyclopentyliden oder Cyclohexyliden.

Hydrolytisch abspaltbare Gruppen $X_2$ und/oder $X'_5$, wie Acylreste von organischen Carbonsäuren, z.B. Niederalkanoyl oder Halogen-niederalkanoyl, Carbamoyl, oder Halbester der Kohlensäure, z.B. Niederalkoxycarbonyl, ferner z.B. Tritylreste, sowie durch die Reste $X_2$ und $X'_5$ zusammen gebildete Niederalkyliden-, 1-Phenyl-niederalkyliden- oder Cycloalkylidengruppen oder durch die Reste $X_2$ und $X'_5$ zusammen gebildete zweibasische Acylreste, wie Phthaloyl oder die Carbonylgruppe können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder in Gegenwart von Ammoniak oder eines Amins, wie Isopropylamin, oder Hydrazinhydrat, abgespalten werden.

Acidolytisch abspaltbare Reste $X_2$ und/oder $X_3'$ sind insbesondere gewisse Acylreste von Halbestern der Kohlensäure, wie z.B. tert.- Niederalkoxycarbonyl oder gegebenenfalls substituierte Diphenylmethoxycarbonylreste, ferner auch tert.-Niederalkylreste, z.B. tert.-Butyl; diese können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig in Gegenwart eines aktivierenden Mittels, wie Anisol), sowie mit Ameisensäure abgespalten werden.

Die obigen Reaktionen werden in üblicher Weise und in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/ oder in der Atmosphäre eines Inertgases, z.B. Stickstoff.

Die Ausgangsstoffe der Formel II können, sofern sie neu sind, nach an sich bekannten Methoden, gegebenenfalls in situ, hergestellt werden.

So kann man z.B. ein Ausgangsmaterial der Formel II mit der Gruppe IIb als $X_1$ erhalten, indem man eine Verbindung der Formel

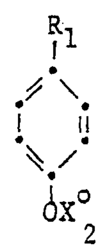

(III),

worin $X_2^o$ z.B. mittels saurer Mittel, etwa konz. Mineralsäure, wie konz. Bromwasserstoffsäure, oder Aluminiumchlorid abspaltbares Niederalkyl, z.B. Methyl ist, mit einem reaktionsfähigen Derivat der Essigsäure, z.B. Acetylchlorid unter den Bedingungen nach Friedel-Crafts zu einer Verbindung der Formel

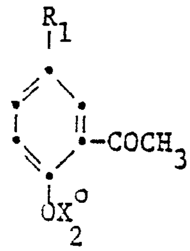

(IV),

umsetzt, diese Verbindung mittels eines geeigneten Oxidationsmittels, etwa Natriumhypochloritlösung, zur Verbindung der Formel

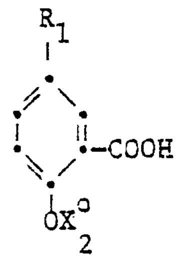

(V)

oxidiert, anschliessend diese Verbindung auf übliche Weise, z.B. über das mittels Thionylchlorid erhaltene Säurechlorid und dessen Umsetzung mit einem Amin der Formeln $H_2N-X_3$ oder $H_2N-X_3'$ in die eine Carbamoylgruppe enthaltende Verbindung der Formel

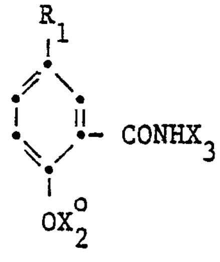

(VI)

bezw. der Formel

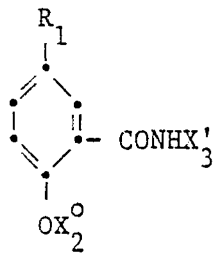

(VIa)

überführt, anschliessend zur Herstellung eines Ausgangsmaterials der Formel II mit der Gruppe IIa eine Verbindung der Formel VI durch Reduktion, z.B. mittels eines Hydridreduktionsmittels, wie etwa Lithiumaluminiumhydrid, in eine die Gruppe IIa enthaltende Verbindung der Formel

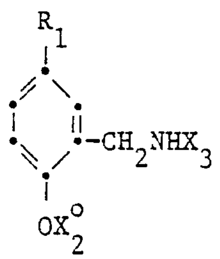

(VIb)

umwandelt, anschliessend in einer Verbindung der Formeln VI, VIa oder VIb die Gruppe $-OX_2^o$ in die Hydroxygruppe z.B. mittels saurer Mittel, wie z.B. Aluminiumchlorid oder einer konzentrierten Mineralsäure, wie konzentrierter Bromwasserstoffsäure, überführt, die erhaltene die freie Hydroxygruppe enthaltende Verbindung VII anschliessend zu einem Ausgangsmaterial der Formel II halogeniert, wobei zur Einführung von Chlor z.B. elementares Chlor oder Sulfurylchlorid, von Brom oder Jod z.B. elementares oder in Form eines reaktionsfähigen Derivats, z.B. Brommonochlorid oder Jodmonochlorid, vorliegendes Brom oder Jod, oder letzteres als Lösung in Kaliumjodid verwendet werden kann. Falls ungesättigte Reste $R_1$ vorliegen, empfiehlt sich zur Einführung z.B. von Brom oder Jod ein reaktionsfähiges Derivat davon, etwa wie beschrieben, zu verwenden.

Ein Ausgangsmaterial der Formel II mit einer Gruppe IIe als $X_1$ ist durch Umwandlung der Gruppe $-CONHX_3'$, worin $X_3'$ für Wasserstoff steht, in einer Verbindung der Formel VIa oder VII in die Gruppe $-C{\equiv}N$ und die sich anschliessende, z.B. analog wie beschrieben, durchzuführende Reaktionsfolge zugänglich. Die Umwandlung wird durch Abspaltung von Wasser auf übliche Weise, z.B. mittels wasserentziehender Mittel, wie Thionylchlorid oder Phosphorpentachlorid oder einem Carbodiimid, z.B. Cyclohexylcarbodiimid vorgenommen. Ein Ausgangsmaterial der Formel II mit der Gruppe IIc oder IId ist durch Umsetzung eines entsprechenden Aldehyds oder dessen reaktionsfähigen Derivaten wie einem Acetal, etwa dem Diäthylacetal, der Formel

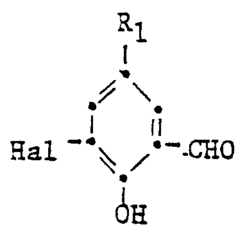

(VIII),

mit einer Verbindung der Formel $H_2N-X_4$ oder mit Hydroxylamin zugänglich, wobei solche Ausgangsstoffe auch in situ hergestellt werden können. Eine Verbindung der Formel (VIII) wiederum kann durch Einführen der Gruppe -CHO in ein entsprechend substituiertes o-Halogenphenol, z.B. mittels Chloroform in alkalischem Medium oder durch Umsetzung mit Hexamethylentetramin in Gegenwart von Borsäure oder einem Aethoxyäthanol-Borsäure-Glyceringemisch erhalten werden. Diese Umsetzungen werden auf übliche Weise vorgenommen.

Ein Ausgangsmaterial der Formel II mit der Gruppe IIf oder IIg kann durch Umsetzung einer Verbindung der Formel

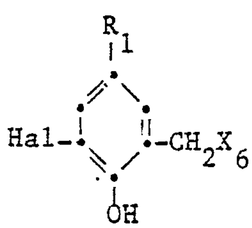

(IX),

worin $X_6$ für eine reaktionsfähige veresterte Hydroxygruppe, z.B. eine Methansulfonyloxygruppe steht, oder Halogen, insbesondere Chlor, Brom oder Jod bedeutet, mit einem Salz der Stickstoffwasserstoffsäure, insbesondere einem Metallsalz, etwa Natrium- oder Kaliumazid, bzw. einem Metallsalz der salpetrigen Säure, z.B. Silbernitrit oder Kaliumnitrit, erhalten werden. Die Umsetzungen erfolgen in üblicher Weise; z.B. erfolgt die Umsetzung mit Natrium- oder Kaliumazid in einem polaren Lösungsmittel, etwa einem Niederalkanol, z.B. Aethanol, oder einem gegebenenfalls N-niederalkylierten Fettsäureamid, wie z.B. Dimethylformamid oder N-Methylacetamid. Die Umsetzung z.B. mit Silbernitrit erfolgt auf bekannte Weise in einem Halogen-, z.B. einem Chlorkohlenwasserstoff, wie Methylenchlorid, während man z.B. Kaliumnitrit in einem polaren Lösungsmittel, z.B. Acetonitril, vorzugsweise in Gegenwart eines Katalysators, etwa eines Kronenäthers wie etwa 18-Krone-6, oder Natriumnitrit in einem niederen Fettsäureamid, z.B. Dimethylformamid, zur Umsetzung mit einer Verbindung der Formel (IX) bringt. Diese Umsetzungen werden in an sich bekannter Weise vorgenommen.

Ein Ausgangsmaterial der Formel IX wiederum wird erhalten, indem man in einer Verbindung der Formel

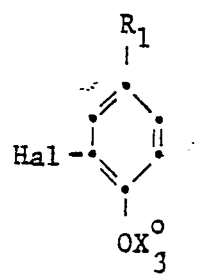

(XI)

worin $X_3^o$ eine z.B. mittels saurer Mittel, wie Aluminiumchlorid, abspaltbare und durch Wasserstoff ersetzbare Gruppe, wie Niederalkyl, z.B. Methyl, oder eine Acylgruppe, wie die Acetylgruppe bedeutet,

BAD ORIGINAL

die Gruppe der Formel $-CH_2X_6$ (X) einführt. Diese Reaktion kann man durch Umsetzung der Verbindung der Formel (XI) mit Formaldehyd in alkalischem Medium, z.B. in wässriger Natriumhydroxid- oder Natriumbicarbonatlösung und Umsetzung der erhaltenen Hydroxymethylverbindung z.B. mit Methansulfonylchlorid in Pyridin zur entsprechenden Methansulfonyloxyverbindung bewirken. Oder man setzt eine Verbindung der Formel (XI) mit Formaldehyd und einem Halogenwasserstoff, insbesondere Chlor- oder Bromwasserstoff in Gegenwart eines Kondensationsmittels, z.B. Zinkchlorid, zur entsprechenden Halogenmethyl-Verbindung um und wandelt anschliessend die Gruppe der Formel $-OX_3^o$ z.B. mittels Hydrolyse in Gegenwart saurer Mittel, etwa wie oben beschrieben, in die Hydroxygruppe um. Anstelle von Formaldehyd kann auch ein reaktionsfähiges Derivat davon, z.B. ein Acetal, wie etwa Formaldehyddimethylacetal, eingesetzt werden. Schliesslich kann die Einführung z.B. einer Chlormethylgruppe auch mittels Chlormethyläther erfolgen.

Ein Ausgangsstoff der Formel II mit der Gruppe IIh kann durch Umsetzung einer Verbindung der Formel

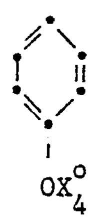

(XII),

worin $X_4^o$ für Wasserstoff steht oder eine, z.B. mittels saurer Mittel, etwa wie oben beschrieben, abspaltbare und durch Wasserstoff ersetzbare Gruppe darstellt, mit einem dem Rest $R_1$ entsprechenden Alkohol unter sauren Bedingungen, etwa nach Friedel-Crafts, umsetzt, die erhaltene Verbindung der Formel

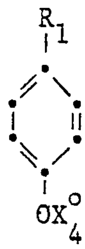

(XIII),

z.B. wie oben beschrieben halogeniert, und die erhaltene Verbindung der Formel

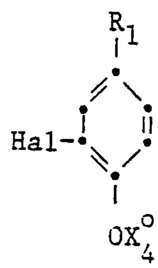

(XIV)

mit einer Verbindung der Formel $HOCH_2-NX_5X'_5$ (XV), umsetzt. Diese Umsetzung wird in üblicher Weise in saurem Medium, z.B. in einer Niederalkancarbonsäure, etwa Essigsäure, oder einem Gemisch derselben mit einer Mineralsäure, wie konz. Schwefelsäure vorgenommen. In einer auf diese Weise erhaltenen Verbindung der Formel

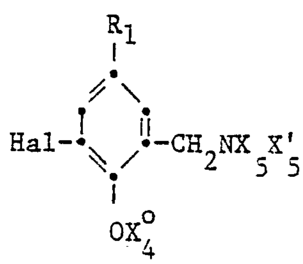

(XVI)

kann anschliessend die gegebenenfalls geschützte Hydroxygruppe mittels saurer Mittel, etwa Aluminiumchlorid z.B. wie oben beschrieben in die freie Hydroxygruppe umgewandelt werden.

Diese Umsetzungen werden in an sich bekannter Weise vorgenommen.

Die neuen Verbindungen der Formel I können ebenfalls erhalten werden, indem man in einer Verbindung der Formel

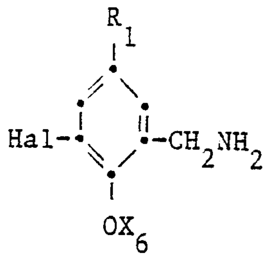

(XVII),

worin $X_6$ einen abspaltbaren und durch Wasserstoff ersetzbaren Rest bedeutet, oder in einem Salz davon, den Rest $X_6$ abspaltet und durch Wasserstoff ersetzt und gewünschtenfalls die zusätzlichen Verfahrensschritte durchführt.

Ein abspaltbarer und durch Wasserstoff ersetzbarer Rest $X_6$ ist z.B. ein mittels Reduktion, einschliesslich Hydrogenolyse oder durch Solvolyse einschliesslich Hydrolyse, Acidolyse oder Alkoholyse abspaltbarer Rest.

Ein hydrogenolytisch abspaltbarer Rest $X_6$ ist in erster Linie eine α-Arylniederalkylgruppe, wie eine gegebenenfalls substituierte 1-Phenylniederalkylgruppe, worin Niederalkyl bis zu 7 Kohlenstoffatome hat und worin Substituenten, insbesondere des Phenylteils, z.B. Niederalkyl oder Niederalkoxy mit jeweils bis zu 7 Kohlenstoffatomen, z.B. Methyl oder Methoxy, sein können, und ganz besonders Benzyl.

Die reduktive Abspaltung des Restes $X_6$ kann insbesondere z.B. durch Behandeln mit katalytisch aktiviertem Wasserstoff, wie Wasserstoff in Gegenwart eines geeigneten Hydrierkatalysators z.B. eines Nickel-, Platin- oder Palladium-, aber auch eines Rhodium- oder Rutheniumkatalysators erfolgen. Oder man arbeitet mit einem geeigneten Hydridreduktionsmittel, wie Diboran oder einem Alkalimetallborhydrid, z.B. Natriumhydrid oder Natriumcyanoborhydrid.

Unter reduktiv abspaltbaren Resten $X_6$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl, wie 2,2,2-Trichloräthoxycarbonyl, die z.B. beim Behandeln mit einem reduzierenden Schwermetall, wie Zink, oder mit einem reduzierenden Schwermetallsalz, wie einem Chrom(II)-salz, z.B. -chlorid oder -acetat, üblicherweise in Gegenwart einer organischen Carbonsäure, wie Ameisensäure oder Essigsäure, und von Wasser abgespalten werden können.

Die obigen Reduktionsreaktionen werden in an sich bekannter Weise, üblicherweise in Gegenwart eines inerten Lösungsmittels, und, wenn notwendig, unter Kühlen oder Erwärmen, z.B. in einem Temperaturbereich von etwa -20° bis etwa + 150°, und/oder in einem geschlossenen Gefäss unter Druck und/oder in einer Inertgas-, z.B. Stickstoffatmosphäre, durchgeführt.

Gegebenenfalls vorhandene, unter den Bedingungen der beschriebenen Reduktionsmethoden in die Gruppe Ia überführbare, in der Formel (II) definierte Reste $X_1$, z.B. solche der oben beschriebenen Formeln IIa, IIb, IIc, IId, IIf oder IIg können gleichzeitig in die Gruppe Ia, und/oder gegebenenfalls vorhandene unter den Bedingungen der beschriebenen Reduktionsmethoden der Umwandlung in gesättigte Reste $R_1$ zugängliche ungesättigte Reste $R_1$ gleichzeitig in gesättigte Reste $R_1$ umgewandelt werden.

Ein mittels Solvolyse wie Hydrolyse, Acidolyse oder Alkoholyse abspaltbarer und durch Wasserstoff ersetzbarer Rest $X_6$ ist z.B., ebenso wie eine wie oben beschrieben, solvolytisch wie hydrolytisch, acidolytisch oder alkoholytisch spaltbare und durch Wasserstoff ersetzbare Gruppe $X_5'$ insbesondere ein Acylrest, wie der Acylrest einer organischen Carbonsäure, z.B. Niederalkanoyl, wie Acetyl, Halogenniederalkanoyl, wie Halogenacetyl, z.B. Chloracetyl, oder Carbamoyl, oder Aroyl, wie Benzoyl, ferner der Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie des Phenylteils, z.B. die oben gegebenen Bedeutungen haben, und in erster Linie Trityl, darstellt. Eine auf diese Weise abspaltbare Gruppe $X_6$ kann ausserdem Niederalkyl, z.B. Methyl, oder eine 1-Phenylniederalkylgruppe wie gegebenenfalls, z.B. wie oben definiert substituiertes Benzyl sein.

Hydrolytisch abspaltbare Reste $X_6$, wie Acylreste von organischen Carbonsäuren, z.B. Niederalkanoyl oder Halogenniederalkanoyl, Carbamoyl, oder Halbester der Kohlensäure, z.B. Niederalkoxycarbonyl, ferner z.B. Tritylreste, können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxides oder -carbonats oder in Gegenwart von Ammoniak oder eines Amins, wie Isopropylamin oder Hydrazinhydrat, abgespalten werden.

Acidolytisch abspaltbare Reste $X_6$ sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch tert.-Niederalkylreste, z.B. tert.-Butyl; diese können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäuren (wenn notwendig in Gegenwart eines aktivierenden Mittels, wie Anisol) sowie mit Ameisensäure abgespalten werden.

Gegebenenfalls vorhandene, mittels der beschriebenen Methoden der Solvolyse, wie Hydrolyse, Acidolyse oder Alkoholyse in die Gruppe Ia überführbare, in der Formel II definierte Reste $X_1$, z.B. solche der oben erläuterten Formel IIh, können auf diese Weise gleichzeitig in die Gruppe Ia umgewandelt werden.

Die obigen Reaktionen werden in üblicher Weise und in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Die Ausgangsstoffe der Formel XVII können, sofern sie neu sind, nach an sich bekannten Methoden, gegebenenfalls in situ, hergestellt werden.

So kann man z.B. analog den oben für die Herstellung von Ausgangsstoffen der Formeln XII - XIV beschriebenen Verfahren eine Verbindung der Formel

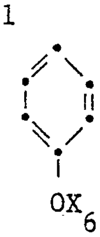

(XVIII)

mit einem dem Rest $R_1$ entsprechenden Alkohol unter sauren Bedingungen, etwa nach Friedel-Crafts, in üblicher Weise umsetzen, und die erhaltene Verbindung der Formel

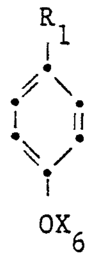

(XIX)

nach bekannten Methoden in einem geeigneten Lösungsmittel, wie Eisessig, oder einem Halogenkohlenwasserstoff, wie Chloroform oder Tetrachlorkohlenstoff, halogenieren und in die erhaltene Verbindung der Formel

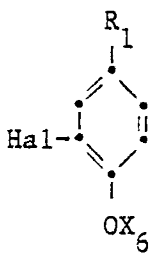

(XX)

z.B. analog, wie oben beschrieben, die Gruppe der Formel X einführen, und in der erhaltenen Verbindung der Formel

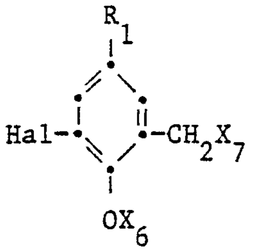

(XXI)

z.B. wie oben beschrieben, den Rest $X_7$ in die primäre Aminogruppe umwandeln.

Ein weiterer zur Herstellung der Verbindung der Formel XVII geeigneter Ausgangsstoff kann erhalten werden, indem man die Verbindung der Formel XX mit einer Verbindung der Formel XV, z.B. wie oben beschrieben zur Verbindung der Formel

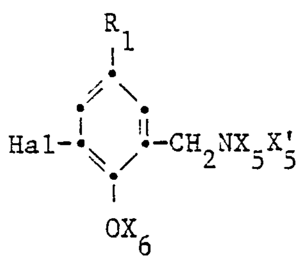

(XXII),

umsetzt.

Diese Reaktionen werden in üblicher Weise vorgenommen.

Die neuen Verbindungen der Formel I können ebenfalls erhalten werden, indem man eine Verbindung der Formel

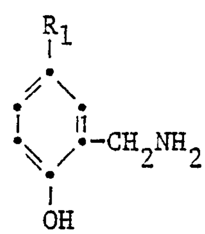

(XXIII)

halogeniert, und, wenn erwünscht, die zusätzlichen Verfahrensschritte durchführt.

So kann man z.B. für die Einführung von Chlor, Brom oder Jod diese in elementarer Form, letzteres auch in Form einer Lösung von Jod in Kaliumjodid, verwenden, oder man setzt für die Einführung von Chlor oder Brom Sulfurylchlorid oder Sulfurylbromid ein.

Für die Einführung von Halogen kann auch ein reaktionsfähiges Derivat davon, z.B. eine geeignete Dihalogenverbindung, wie Brommonochlorid oder Jodmonochlorid, verwendet werden. Diese

Methode wird vorzugsweise dann angewendet, wenn $R_1$ einen ungesättigten Rest darstellt.

Zweckmässigerweise werden diese Umsetzungen in einem Lösungsmittel, etwa in saurem Medium, z.B. in einer Niederalkancarbonsäure, z.B. Essigsäure, vorgenommen, falls elementares Chlor oder Brom verwendet wird, während die Einführung von Brom oder Jod unter Verwendung eines reaktionsfähigen Derivats davon, z.B. in wässriger Chlorwasserstoff- oder Bromwasserstofflösung vorgenommen werden kann. Diese Umsetzungen werden in an sich bekannter Weise durchgeführt.

Zur Herstellung eines Ausgangsmaterials der Formel XXIII kann man z.B. so vorgehen, dass man in einer Verbindung der Formel

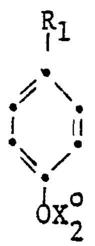

(XXIV),

worin $X_2^o$ eine z.B. mittels saurer Mittel, etwa wie oben beschrieben, abspaltbare und durch Wasserstoff ersetzbare Gruppen bedeutet, die oben erläuterte Gruppe der Formel X, z.B. wie beschrieben, einführt, und die z.B. Chlor oder Brom darstellende Gruppe $X_7$ gegen die primäre Aminogruppe auf übliche Weise austauscht, z.B. durch Umsetzung mit Hexamethylentetramin in einem organischen Lösungsmittel, wie Chloroform, und Spalten des erhaltenen Addukts mit wässeriger Mineralsäure, z.B. verdünnter Salzsäure, oder durch Umsetzen mit einer Phthalimidverbindung, z.B. Phthalimidkalium und Hydrolysieren der N-Phthalimino-Verbindung mit einer Säure, z.B. konzentrierter Salzsäure, oder mittels Hydrazinhydrat.

Die neuen Verbindungen der Formel I können ebenfalls erhalten werden, indem man in einer Verbindung der Formel

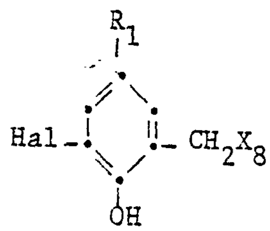

(XXV),

worin $X_8$ für eine reaktionsfähige veresterte Hydroxygruppe steht, die Gruppe $X_8$ gegen die primäre Aminogruppe austauscht, und, wenn erwünscht, die zusätzlichen Verfahrensschritte durchführt.

Eine reaktionsfähige veresterte Hydroxygruppe $X_8$ ist eine, durch eine starke Säure, insbesondere eine starke anorganische Säure, wie eine Halogenwasserstoffsäure, insbesondere Chlor-, Brom oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Der Austausch der Gruppe $X_8$ gegen die primäre Aminogruppe kann z.B. durch Umsetzung mit einer die primäre Aminogruppe liefernden Verbindung, z.B. mit Hexamethylentetramin, bewerkstelligt werden. Dieses wird z.B. mit einer Verbindung der Formel XXV in einem organischen Lösungsmittel, z.B. einem Halogenkohlenwasserstoff, wie Chloroform, zum entsprechenden Addukt umgesetzt, welches anschliessend in saurem Medium, z.B. mittels wässeriger Mineralsäure, etwa verdünnter Salzsäure, in eine Verbindung der Formel I umgewandelt wird. Man kann ferner eine Verbindung der Formel XXV mit einer geeigneten Phthalimidverbindung, z.B. mit Phthalimidkalium in einem geeigneten Lösungsmittel, z.B. einem solchen polaren Charakters, wie einem Niederalkanol, z.B. Aethanol, oder einem gegebenenfalls N-alkylierten Fettsäureamid, z.B. Dimethylformamid oder N-Methylacetamid, zur entsprechenden N-Phthalimidoverbindung umsetzen, aus welcher dann, z.B. wie oben beschrieben, mittels Hydrolyse, etwa mit einer Mineralsäure, wie konz. Salzsäure, oder mittels Hydrazinhydrat die Verbindung der Formel I erhalten wird. Diese Umsetzungen werden in üblicher Weise vorgenommen.

Ein Ausgangsstoff der Formel XXV kann in an sich bekannter Weise erhalten werden, indem man in einer Verbindung der Formel

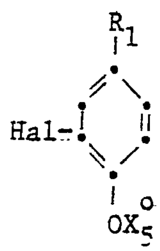

(XXVI)

worin $X_5^o$ Wasserstoff ist oder eine abspaltbare durch Wasserstoff ersetzbare Gruppe, z.B. eine Niederalkylgruppe wie Methyl, oder eine Acylgruppe, wie eine Niederalkanoylgruppe z.B. Acetyl, darstellt, die Gruppe der Formel $-CH_2X_8$ (XXVII), einführt, und einen von Wasserstoff verschiedenen Rest $X_5^o$ z.B. mittels saurer Mittel, wie Aluminiumchlorid, oder mittels einer Säure, wie einer Mineralsäure z.B. Schwefelsäure, abspaltet und durch Wasserstoff ersetzt. Die Einführung der Gruppe der Formel XXVII kann, z.B. wie oben beschrieben, mittels Formaldehyd in alkalischem Medium und anschliessender Umwandlung der Hydroxygruppe in eine der Gruppe $X_8$ entsprechende reaktionsfähige veresterte Hydroxygruppe in üblicher Weise, z.B. wie oben beschrieben, erfolgen. Oder man setzt eine Verbindung der Formel XXVI, worin die Hydroxygruppe vorzugsweise, etwa wie angegeben, geschützt ist, mit Formaldehyd und Halogenwasserstoff, etwa Chlor- oder Bromwasserstoff in Gegenwart eines Kondensationsmittels, wie Zinkchlorid, zur entsprechenden Halogenmethylverbindung um,und überführt eine gegebenenfalls vorhandene, z.B. wie angegeben, geschützte Hydroxygruppe in die freie Hydroxygruppe. Die Einführung einer der Formel XXVII entsprechenden Chlormethylgruppe kann auch mittels Chlormethyläther erfolgen. Diese Umsetzungen werden in an sich bekannter Weise vorgenommen.

Eine erhaltene Verbindung der Formel I kann in üblicher Weise in einen anderen Endstoff der Formel I überführt werden, indem man z.B. einen ungesättigten Rest $R_1$ zu einem gesättigten Rest $R_1$ reduziert und, wenn erwünscht, die zusätzlichen Verfahrensschritte durchführt.

Die Reduktion von ungesättigten Resten $R_1$ kann in üblicher Weise, z.B. mittels katalytisch aktiviertem Wasserstoff in Gegenwart eines Hydrierkatalysators, z.B. eines Nickel-, Platin- oder Palladiumkatalysators durchgeführt werden. Diese Reduktionsreaktionen werden in an sich bekannter Weise, üblicherweise in Gegenwart eines inerten Lösungsmittels, und wenn notwendig, unter Kühlen oder Erwärmen z.B. in einem Temperaturbereich von etwa -20° bis etwa +150° und/oder in einem geschlossenen Gefäss unter Druck und/oder in einer Inertgas-, z.B. Stickstoffatmosphäre durchgeführt.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon gegebenenfalls auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln,

wie Alkalimetallhydroxiden,-carbonaten oder -hydrogencarbonaten oder Ionenaustauschern, in die freien Verbindungen überführt werden. Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch annehmbaren, nicht toxischen Salzen eignen.

Diese und andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach Wahl der Ausgangsstoffe und Arbeitsweisen als Racemate oder optische Antipoden oder, sofern sie mindestens zwei asymmetrische Kohlenstoffatome enthalten, auch als Racematgemische vorliegen.

Erhaltene Racematgemische können auf Grund der physikalischchemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Säure und Trennen des auf diese Weise erhaltenen Salzgemisches, z.B. auf Grund von verschiedener Löslichkeiten, in die diastereomeren Salze, aus denen die freien Antipoden durch Einwirkung geeigneter Mittel freigesetzt werden können.

Besonders gebräuchliche, optisch aktive Säuren sind z.B. die D- und L-Formen von Weinsäure, OO'-Di-(p-Toluolyl)-Weinsäure, Aepfelsäure, Mandelsäure, Camphersulfonsäure, Glutaminsäure, Asparaginsäure oder Chinasäure.

Vorteilhafterweise isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe können auch als optische Antipoden eingesetzt werden.

Die Ausgangsstoffe sind bekannt oder können, falls sie neu sind, nach an sich bekannten Methoden, wie oben, z.B. analog wie in dem Beispiel, beschrieben, erhalten werden. Neue Ausgangsstoffe bilden ebenfalls einen Gegenstand der Erfindung. Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen enthalten, die sich zur enteralen, z.B. oralen oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium- oder Calciumstearat, und/oder Polyäthylenglykol, enthalten. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer enthalten, Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50% Lyophilisate bis zu 100% des Aktivstoffes.

Die Dosierung kann von verschiedenen Faktoren, wie Applikationsweise, Spezies, Alter und/oder individuellem Zustand abhängen. So liegen die täglich zu verabreichenden Dosen bei oraler Applikation an Warmblütern von etwa 70 kg Körpergewicht vorzugsweise zwischen etwa 0,05 und 2,0 g.

Die folgenden Beispiele dienen zur Illustration der Erfindung; Temperaturen werden in Celsiusgraden angegeben.

Beispiel 1: Zu 230 ml Essigsäure werden bei 15° unter Rühren während 20 Minuten 25 ml konz. Schwefelsäure zugetropft. Man rührt noch 10 Minuten nach und tropft dann 39 g 2-Brom-4-(1-methyl-1-cyclopentyl)-phenol zu. Nach weiteren 10 Minuten Rühren bei Raumtemperatur fügt man 21 g N-Hydroxymethyl-chloracetamid zu der klaren Lösung und rührt das Gemisch während 18 Stunden bei Raumtemperatur. Anschliessend giesst man das Gemisch auf 2 Liter Eiswasser und extrahiert das ausgefallene schmierige Produkt 2-mal mit Aether. Die vereinigten organischen Phasen werden über Natriumsulfat getrocknet und eingedampft. Das zurückbleibende schwach gelbliche Oel wird in 640 ml Aethanol gelöst, mit 127 ml konz. Salzsäure versetzt und das Gemisch 16 Stunden unter Rückflusskochen gerührt. Danach engt man das Reaktionsgemisch auf die Hälfte seines Volumens ein und kühlt ab. Die ausgefallenen Kristalle werden abgesaugt, mit wenig kaltem Aethanol, dann mit Aether gewaschen und schliesslich aus Aethanol/Aether unter Zusatz von Aktivkohle umkristallisiert. Man erhält das Hydrochlorid des 2-Aminomethyl-4-(1-methyl-1-cyclopentyl)-6-bromphenol als weisses Kristallisat vom Smp. 221°.

Das als Ausgangsstoff verwendete 2-Brom-4-(1-methyl-1-cyclopentyl)-phenol kann auf folgende Weise hergestellt werden:

a) Ein Gemisch von 263 g Phenol und 80 g 1-Methyl-cyclopentanol wird unter Rühren bei 40°C vorgelegt. Dazu gibt man unter Eiskühlung 106 g Aluminiumchlorid in vier Portionen, wobei Aufschäumen eintritt. Danach wird das Gemisch unter langsamem Rühren im Laufe von zwei Stunden auf 80°C erwärmt, lässt noch weitere 6 Stunden bei 80° reagieren und rührt dann das Gemisch in 1 Liter Eiswasser ein. Nach 30 Minuten Rühren extrahiert man erschöpfend mit Aether, trocknet die vereinigten organischen Phasen über Natriumsulfat und dampft ein. Der Rückstand wird bei 100°/1 mm Torr fraktioniert destilliert, wonach man das 4-(1-Methyl-1-cyclopentyl)-phenol erhält, welches beim Stehenlassen kristallisiert; Smp. 84-88°.

b) Ein Gemisch von 114,4 g 4-(1-Methyl-1-cyclopentyl)-phenol, 162 ml Chloroform und 162 ml Tetrachlorkohlenstoff wird unter Rühren bei 0-5°C vorgelegt. Dazu tropft man innerhalb 40 Minuten eine Lösung von 104 g Brom in 130 ml Chloroform und rührt noch eine Stunde bei 0-5° nach. Danach dampft man die rote Lösung unter vermindertem Druck bei ca. 50° Badtemperatur ein, versetzt den Rückstand mit 3,8 g Natriumcarbonat, der dann im Hochvakuum bei 120°/0,1 mm Hg fraktioniert destilliert wird, wonach man das 2-Brom-4-(1-methyl-1-cyclopentyl)-phenol vom Kp. 120°/01 mm Hg erhält.

Beispiel 2: Analog der im Beispiel 1 beschriebenen Verfahrensweise erhält man unter Verwendung von 36,85 g 2-Brom-4-cyclopentylphenol das 2-Aminomethyl-4-cyclopentyl-6-bromphenol vom Smp. 246-248°.

Das als Ausgangsmaterial benötigte 2-Brom-4-cyclopentylphenol wird erhalten, indem man analog Beispiel 1a) unter Verwendung von 63,6g Cyclopentanol das 4-Cyclopentylphenol vom $Kp_{18}$ 150-155° herstellt, und dieses analog Beispiel 1b) zum 2-Brom-4-cyclopentylphenol bromiert; $Kp_{1}$ 135-141°.

Beispiel 3: Ein Gemisch von 20,5 g 2-Aminomethyl-4-(1-methyl-1-cyclopentyl)-phenol, 30 ml Chloroform und 30 ml Tetrachlorkohlenstoff wird unter Rühren bei 0-5°C vorgelegt. Dazu tropft man innerhalb 40 Minuten eine Lösung von 15 g Brom in 130 ml Chloroform und rührt noch eine Stunde bei 0-5° nach. Danach dampft man die Lösung im Vakuum bei ca. 50° Badtemperatur ein und verteilt den Rückstand zwischen Chloroform und Ammoniak. Das Chloroform wird über Natriumsulfat getrocknet, verdampft und das zurückbleibende Oel mit äthanolischer Salzsäure in das Hydrochlorid überführt. Dieses wird unter Zusatz von Aktivkohle aus Aethanol/Aether umkristallisiert, wonach das erhaltene Hydrochlorid des 2-Aminomethyl-4-(1-methyl-1-cyclopentyl)-6-bromphenol bei 221° schmilzt.

Das als Ausgangsstoff verwendete 2-Aminomethyl-4-(1-methyl-1-cyclopentyl)-phenol kann auf folgende Weise hergestellt werden:

Zu 230 ml Essigsäure werden bei 15° unter Rühren während 20 Minuten 25 ml konz. Schwefelsäure zugetropft. Man rührt noch 10 Minuten nach und tropft dann 30 g 4-(1-methyl-1-cyclopentyl)-phenol zu. Nach weiteren 10 Minuten Rühren bei Raumtemperatur fügt man 21 g N-Hydroxymethyl-chloracetamid zu der klaren Lösung und rührt das Gemisch während 18 Stunden bei Raumtemperatur. Anschliessend giesst man das Gemisch auf 2 Liter Eiswasser und extrahiert das ausgefallene schmierige Produkt 2-mal mit Aether. Die vereinigten organischen Phasen werden über Natriumsulfat getrocknet und eingedampft. Das zurückbleibende schwach gelbliche Oel wird in 110 ml Aethanol gelöst, mit 21 ml konz. Salzsäure versetzt und das Gemisch 16 Stunden unter Rückfluss gekocht. Hierauf wird das Reaktionsgemisch zur Trockene eingedampft und der Rückstand zwischen Chloroform und Ammoniak verteilt. Die Chloroformlösung wird über Natriumsulfat getrocknet, verdampft und das rohe 2-Aminomethyl-4-(1-methyl-1-cyclopentyl)-phenol als solches weiterverwendet.

Beispiel 4: Eine Lösung von 3,75 g 1-Aminomethyl-2-benzyloxy-3-brom-5-(1-methyl-1-cyclopentyl)-benzol in 80 ml abs. Aethanol wird in Gegenwart von 0,5 g Palladium-auf-Kohle-Katalysator bei Raumtemperatur hydriert. Dann wird der Katalysator abfiltriert und das Filtrat im Vakuum eingedampft. Das zurückbleibende Oel wird mit aethanolischer Salzsäure in das Hydrochlorid überführt und aus Aethanol/Aether umkristallisiert. Das erhaltene 2-Aminomethyl-4-(1-methyl-1-cyclopentyl)-6-bromphenol-hydrochlorid schmilzt bei 221°.

Dals als Ausgangsprodukt verwendete 1-Aminomethyl-2-benzyloxy-3-brom-5-(1-methyl-1-cyclopentyl)-benzol kann wie folgt hergestellt werden:

17,6 g 4-(1-Methyl-1-cyclopentyl)-phenol werden mit 17,1 g Benzylbromid und 27,6 g Kaliumcarbonat in 200 ml Acetonitril 8 Stunden unter Rückfluss erhitzt. Hierauf wird abfiltriert, das Filtrat im Vakuum eingedampft und die noch anhaftende Feuchtigkeit durch mehrmaliges Behandeln mit Toluol entfernt. Das zurückbleibende 1-Benzyloxy-4-(1-methyl-1-cyclopentyl)-benzol wird ohne weitere Reinigung weiterverarbeitet.

Ein Gemisch von 26,6 g des erhaltenen 1-Benzyloxy-4-(1-methyl-1-cyclopentyl)-benzol, 30 ml Chloroform und 30 ml Tetrachlorkohlenstoff wird vorgelegt. Zu dem gerührten und auf 0-5° gekühlten Gemisch tropft man innerhalb 40 Minuten eine Lösung von 16 g Brom in 130 ml Chloroform und rührt noch eine Stunde bei 0-5°C nach. Danach dampft man die Lösung im Vakuum bei 50°C Badtemperatur ein, und verteilt den Rückstand zwischen Chloroform und Ammoniak. Die Chloroformschicht wird getrocknet, verdampft und das zurückbleibende 1-Benzyloxy-2-brom-4-(1-methyl-1-cyclopentyl)-benzol destilliert. $Kp_{0,1}$: 140-145°. Zu 80 ml Essigsäure werden bei 15°C unter Rühren während 20 Minuten 8 ml konz. Schwefelsäure zugetropft. Man rührt noch 10 Minuten nach und tropft dann 17,3 g 1-Benzyloxy-2-brom-4-(1-methyl-1-cyclopentyl)-benzol zu. Nach weiteren 10 Minuten fügt man zu der klaren Lösung 7 g N-Hydroxymethyl-chloracetamid und rührt das Gemisch bei Raumtemperatur während 18 Stunden. Dann wird das Gemisch in 2000ml Eiswasser eingetragen und das ausgefallene Produkt mit Methylenchlorid extrahiert. Die organische Phase wird getrocknet, das Lösungsmittel abgedampft, das zurückbleibende Oel in 210 ml Aethanol gelöst und mit 43 ml konz. Salzsäure 16 Stunden unter Rückfluss erhitzt. Hierauf wird das Reaktionsgemisch im Vakuum eingedampft und das zurückbleibende 1-Aminomethyl-2-benzyloxy-3-brom-5-(1-methyl-1-cyclopentyl)-benzol mittels Toluol von noch anhaftender Feuchtigkeit befreit. Das erhaltene Rohprodukt wird als solches weiterverarbeitet.

Beispiel 5: 15,2 g 2-Chlormethyl-4-(1-methyl-1-cyclopentyl)-6-bromphenol werden mit 150 ml methanolischem Ammoniak im geschlossenen Gefäss 8 Stunden auf 150° erhitzt. Dann wird die Lösung im Vakuum verdampft, der Rückstand zwischen Ammoniak und Chloroform verteilt, die organische Phase über Natriumsulfat getrocknet und verdampft. Das zurückbleibende Oel wird mit äthanolischer Salzsäure in das Hydrochlorid überführt, und unter Zusatz von Aktivkohle aus Aethanol/Aether umkristallisiert, wonach das erhaltene 2-Aminomethyl-4-(1-methyl-1-cyclopentyl)-6-bromphenol-hydrochlorid den Smp. 221° zeigt.

Das als Ausgangsprodukt verwendete 2-Chlormethyl-4-(1-methyl-1-cyclopentyl)-6-bromphenol wird wie folgt synthetisiert:

25,5 g 2-Brom-4-(1-methyl-1-cyclopentyl)-phenol, 180 ml konz. Salzsäure, 1,4 ml konz. Schwefelsäure und 21,0 g Methylal werden während 5 Stunden unter Einleiten von Salzsäuregas auf 70° erwärmt. Hierauf wird abgekühlt, das 2-Chlormethyl-4-(1-methyl-1-cyclopentyl)-6-bromphenol abgesaugt und als Rohprodukt weiterverarbeitet.

Beispiel 6: 3,2 g rohes 2-Aminomethyl-4-(1-methyl-2-cyclopenten-1-yl)-6-bromphenol-hydrochlorid werden in 25 ml Methanol in Gegenwart von 0,5 g Rhodium-auf-Kohle-Katalysator bei Normaldruck und Raumtemperatur hydriert. Nach beendeter Wasserstoffaufnahme (1 Moläquivalent) wird vom Katalysator abfiltriert, das Filtrat im Vakuum eingedampft und der Rückstand aus Aethanol/Aether umkristallisiert. Das erhaltene 2-Aminomethyl-4-(1-methyl-1-cyclopentyl)-6-bromphenol-hydrochlorid schmilzt bei 221°.

Das als Ausgangsmaterial benötigte 2-Aminomethyl-4-(1-methyl-2-cyclopenten-1-yl)-6-bromphenol kann wie folgt erhalten werden:

a) Zu einer Mischung von 484 g 2-Bromphenol und 78,4 g 1-Methyl-2-cyclopenten-1-ol gibt man unter Eiskühlung 106 g wasserfreies

Aluminiumchlorid in vier Portionen. Nach beendetem Aufschäumen wird das Reaktionsgemisch im Laufe von zwei Stunden unter langsamem Rühren auf 80° erwärmt, hält noch weitere 6 Stunden bei dieser Temperatur und rührt dann in 1,2 Liter Eiswasser ein und rührt noch 30 Minuten nach. Anschliessend wird das Gemisch erschöpfend mit Aether extrahiert, die vereinigten Aetherlösungen über Natriumsulfat getrocknet und anschliessend zur Trockne verdampft, wonach man 2-Brom-4-(1-methyl-2-cyclopenten-1-yl)-phenol als Rohprodukt erhält.

b) Analog der im Beispiel 1 beschriebenen Arbeitsweise erhält man unter Verwendung von 38,6 g rohem 2-Brom-4-(1-methyl-2-cyclopenten-1-yl)-phenol das 2-Aminomethyl-4-(1-methyl-2-cyclopenten-1-yl)-6-bromphenol, welches in Aethanol gelöst und mit 127 ml konz. Salzsäure versetzt wird. Nach weitgehendem Abdampfen des Lösungsmittels erhält man das Hydrochlorid des 2-Aminomethyl-4-(1-methyl-2-cyclopenten-1-yl)-6-bromphenol, welches als solches weiterverarbeitet wird.

<u>Beispiel 7</u>: Herstellung von 10'000 Tabletten mit einem Gehalt von je 50 mg aktiver Substanz:

<u>Bestandteile</u>:

| | |
|---|---|
| 2-Aminomethyl-4-(1-methyl-1-cyclopentyl)-6-bromphenol-hydrochlorid | 500 g |
| Milchzucker | 1207 g |
| Maisstärke | 75 g |
| Polyäthylenglykol 6000 | 75 g |
| Talkpulver | 75 g |
| Magnesiumstearat | 18 g |
| Gereinigtes Wasser | q.s. |

Verfahren:

Sämtliche festen Bestandteile werden durch ein Sieb von 0,6 mm Maschenweite gesiebt. Der Wirkstoff wird dann mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser zugesetzt. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Patentansprüche:

1. Aromatische Hydroxyverbindungen der Formel

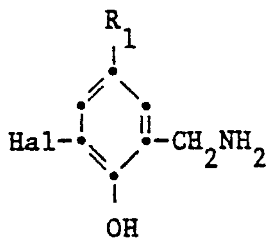

(I),

worin $R_1$ einen cycloaliphatischen Kohlenwasserstoffrest bedeutet und Hal für Halogen steht.

2. Verbindungen der Formel I gemäss Anspruch 1, worin $R_1$ einen gegebenenfalls niederalkylierten Cycloalkyl- oder Cycloalkenylrest mit 5-7 Ringgliedern bedeutet, und Hal für Halogen steht.

3. Verbindungen der Formel I gemäss Anspruch 1, worin $R_1$ einen gegebenenfalls durch Niederalkyl bis und mit 4 Kohlenstoffatomen substituierten Cycloalkyl- oder Cycloalkenylrest mit 5-6 Ringgliedern bedeutet, und Hal für Chlor, Brom oder Jod steht.

4. Verbindungen der Formel I gemäss Anspruch 1, worin $R_1$ einen gegebenenfalls durch Niederalkyl mit und bis 3 Kohlenstoffatomen substituierten Cyclopentyl-, Cyclopentenyl- oder Cyclohexylrest bedeutet, und Hal für Brom oder Jod steht.

5. 2-Aminomethyl-4-(1-methyl-1-cyclopentyl)-6-bromphenol.

6. 2-Aminomethyl-4-cyclopentyl-6-bromphenol.

7. 2-Aminomethyl-4-(1-methyl-2-cyclopenten-1-yl)-6-bromphenol.

8. Verbindungen der Formel I als pharmakologisch aktive Verbindungen.

9. Salze von Verbindungen der Ansprüche 1 bis 7.

10. Pharmazeutisch verwendbare, nicht-toxische Säureadditionssalze von Verbindungen der Ansprüche 1 bis 7.

11. Pharmazeutische Präparate enthaltend eine Verbindung der Ansprüche 1 bis 7 oder 10 zusammen mit einem pharmazeutischen Trägermaterial.

12. Verwendung von pharmazeutischen Präparaten enthaltend eine Verbindung der Ansprüche 1 bis 7 oder 10 als Diuretika und Saluretika.

13. Verwendung von Verbindungen der Formel I zur Bekämpfung von Oedemen und der Hypertonie.

14. Verfahren zur Herstellung von aromatischen Hydroxyverbindungen der Formel

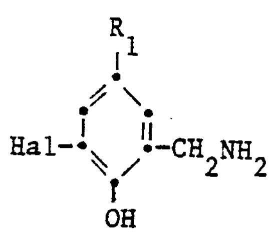

(I),

worin $R_1$ einen cycloaliphatischen Kohlenwasserstoffrest bedeutet und Hal für Halogen steht, dadurch gekennzeichnet, dass man

a) in einer Verbindung der Formel

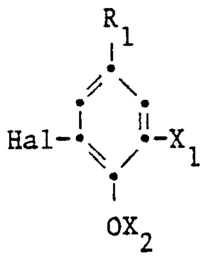

(II),

worin $X_2$ Wasserstoff ist und $X_1$ einen in die Gruppe der Formel $-CH_2NH_2$ (Ia) überführbaren Rest oder $X_1$ und $X_2$ zusammen einen unter Bildung der Hydroxygruppe und der Gruppe Ia abspaltbaren Rest darstellen, oder in einem Salz davon, den Rest $X_1$ in die Gruppe Ia überführt, oder die Reste $X_1$ und $OX_2$ gleichzeitig in die Gruppe Ia bezw. die Hydroxygruppe umwandelt, oder

b) in einer Verbindung der Formel

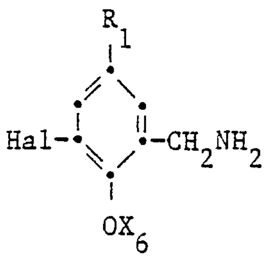

(XVII),

worin $X_6$ einen abspaltbaren und durch Wasserstoff ersetzbaren Rest bedeutet, oder in einem Salz davon, den Rest $X_6$ abspaltet und durch Wasserstoff ersetzt, oder

c) eine Verbindung der Formel

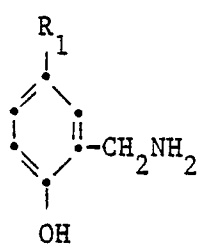

(XXIII)

halogeniert, oder

d) in einer Verbindung der Formel

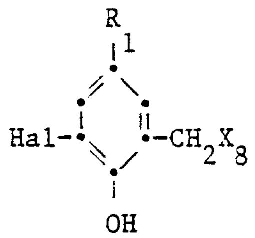

(XXV),

worin $X_8$ für eine reaktionsfähige veresterte Hydroxygruppe steht, die Gruppe $X_8$ gegen die primäre Aminogruppe austauscht, und wenn erwünscht, eine erhaltene Verbindung der Formel I in eine andere Verbindung der Formel I umwandelt, und/oder eine erhaltene freie Verbindung in ein Salz, oder ein erhaltenes Salz in die freie Verbindung überführt, und/oder ein erhaltenes Racemat in die optische Antipoden auftrennt.

Europäisches Patentamt

# EUROPÄISCHER RECHERCHENBERICHT

0020300

Nummer der Anmeldung

EP 80 81 0168

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| X | US - A - 3 794 734 (E.J. CRAGOE und E.M. SCHULTZ)<br>* Patentanspruch 1; Spalte 1, Zeilen 5-8; Beispiel 10 * | 1-4,8-14 | C 07 C 91/30<br>A 61 K 31/135 |
| | DE - A - 2 408 668 (MERCK)<br>* Patentansprüche, Seite 18 * | 1,8-14 | RECHERCHIERTE SACHGEBIETE (Int. Cl.³)<br>C 07 C 91/30<br>A 61 K 31/135 |
| | FR - A - 2 119 066 (MERCK)<br>* Seiten 1-2 * | 1,8-14 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 19-08-1980 | MOREAU |